Europäisches Patentamt

European Patent Office

Office européen des brevets

(11) Numéro de publication: **0 323 429**
**A1**

## (12) DEMANDE DE BREVET EUROPEEN

(21) Numéro de dépôt: 88870195.0

(51) Int. Cl.⁴: **A 61 B 17/58**

(22) Date de dépôt: **22.12.88**

(30) Priorité: 30.12.87 FR 8718540

(43) Date de publication de la demande:
05.07.89 Bulletin 89/27

(84) Etats contractants désignés:
AT BE CH DE ES GB GR IT LI LU NL SE

(71) Demandeur: **Guinounet, Alain**
**7 rue du jardin l'Evêque**
**F-82000 Montauban (FR)**

(72) Inventeur: **Guinounet, Alain**
**7 rue du jardin l'Evêque**
**F-82000 Montauban (FR)**

(74) Mandataire: **Dellicour, Paul**
**Office de Brevets E. Dellicour rue Fabry 18/012**
**B-4000 Liège (BE)**

(54) **Vis de blocage d'une plaque d'ostéosynthèse et procédé de fabrication d'une telle vis.**

(57) L'invention concerne une vis de blocage d'une plaque de matériel d'ostéosynthèse du type de celle comprenant notamment :
- une tige cylindrique filetée 1 destinée à pénétrer dans le tissu osseux du fragment d'une fracture, via une des ouvertures ménagées dans ladite plaque,
- et une tête 2 prenant appui sur la susdite plaque qu'elle maintient plaquée contre ledit fragment.

Selon l'invention, cette vis comporte une tige de préhension 3 qui, associée à la tête 2 de ladite vis coaxialement à cette dernière pour être logée dans le porte-outil de l'arbre de sortie d'un moteur rotatif, est montée dissociable de ladite tête 2 sous l'effet d'un couple de torsion minimum prédéterminé appliqué sur la susdite tige de préhension.

L'invention concerne également un procédé de fabrication d'une telle vis.

Applications : vis de blocage de matériel d'ostéosynthèse.

EP 0 323 429 A1

# Description

## VIS DE BLOCAGE D'UNE PLAQUE D'OSTEOSYNTHESE ET PROCEDE DE FABRICATION D'UNE TELLE VIS

La présente invention a trait au domaine de la chirurgie et concerne plus précisément une méthode de traitement sanglant des fractures, consistant à maintenir les fragments osseux, à l'aide d'appareils métalliques (vis, plaques, etc..) qui permettent la consolidation par formation du cal.

D'une manière générale, le matériel d'ostéosynthèse comprend classiquement des plaques, des vis et des tournevis.

Les plaques d'une part, sont façonnées dans un acier inoxydable austénitique, alliage biocompatible avec le tissu osseux, et selon une taille suffisamment petite pour être tolérée en sous-cutané et par la muqueuse vestibulaire. Des ouvertures sont réparties sur la longueur de la plaque pour autoriser l'introduction de vis.

Les vis d'autre part, dite vis de blocage et également façonnées dans un matériau en acier inoxydable austénitique, comprennent notamment :
- une tige cylindrique filetée auto-taraudeuse destinée à pénétrer dans le tissu osseux du fragment d'une fracture, via une des ouvertures ménagées dans ladite plaque,
- et une tête prenant appui sur la susdite plaque qu'elle maintient plaquée contre ledit fragment.

Ces vis, présentant des caractéristiques prédéterminées (5-9 mm de longueur, 1,6 mm de diamètre de l'âme, 100 de pas, 0,2 mm de profondeur de filet) ont subi quelques améliorations visant notamment à améliorer le couple de torsion, telle par exemple la forme originale de leur tête qui adopte une empreinte cruciforme.

Les tournevis enfin, sont utilisés par paire. Le premier, dit tournevis préhenseur, permet de placer la vis dans le trou préalablement foré et d'amorcer un ou deux tours de spires. Le deuxième, dit tournevis de serrage à lame réversible, se caractérise :
- par un pommeau tournant qui autorise un torsion en maintenant une pression axiale fixe par application de la paume de la main,
- et par un embout à empreinte cruciforme qui, tout en assurant un torsion confortable, permet de désaxer l'axe du tournevis par rapport à celui de la vis selon un angle pouvant aller jusqu'à 25, (désaxement particulièrement avantageux lorsque l'accès est difficile notamment lors d'une intervention dans la cavité buccale).

La technique habituelle de mise en place d'une plaque d'ostéosynthèse s'opère en quatre stades.

Dans un premier stade, la plaque d'ostéosynthèse est choisie en fonction de sa longueur qui doit être suffisante pour autoriser la mise en place de deux vis de blocage de chaque coté du foyer de facture afin d'assurer une contention tridimentionnelle.

Dans un deuxième stade, la plaque est modelée pour s'appliquer intimement contre les refiefs osseux qu'elle contient, afin d'éviter un déplacement secondaire de la réduction lors du torsion des vis (phénomène de rappel).

Dans un troisième stade, des trous sont forés dans le tissu osseux - de préférence en un seul aller-retour pour éviter d'ovaliser ledit trou - à travers les ouvertures de la plaque centrée par rapport au foyer de fracture et maintenue en place, par exemple au moyen d'une pince "kelly" dans un trou adjacent.

Dans un quatrième et dernier stade, il suffit d'engager, à l'aide du tournevis préhenseur, la vis auto-taraudeuse dans le trou de forage, de visser, à l'aide du tournevis de serrage, dans le même axe que ledit trou dont on a gardé en mémoire la direction, et de faire un torsion minimum sans bloquer et en exerçant une contre-pression sur le fragment pour ne pas déplacer la réduction. Si la vis tourne dans le vide, une vis de rattrapage dont l'âme est plus large est alors utilisée. Lorsque toutes les vis sont disposées et vissées, on les bloque sans excès pour éviter l'écrasement du filetage de la vis ainsi que celui de la plaque contre l'os.

Bien que les opérations de forage des trous et de torsion des vis de blocage dans lesdits trous soient des opérations classiques pour le chirurgien, la pose de plaques vissées en traumatologie faciale sont beaucoup plus délicates et exigent de la part de l'homme de l'art, une plus grande dextérité.

Partant de ces constatations, le demandeur a mené des recherches qui ont abouti à la conception d'une vis de blocage permettant d'éliminer certaines manipulations opératoires et offrant en outre de nombreux avantages d'utilisation complémentaires par rapport aux vis de l'art antérieur.

A cet effet, la vis de blocage d'une plaque de matériel d'ostéosynthèse de l'invention, comprenant classiquement une tige cylindrique filetée destinée à pénétrer dans le tissu osseux du fragment d'une fracture et une tête prenant appui sur la susdite plaque, est remarquable en ce qu'elle comporte une tige de préhension qui, associée à la tête de ladite vis coaxialement à cette dernière pour être logée dans le porte-outil de l'arbre de sortie d'un moteur rotatif, est montée dissociable de ladite tête sous l'effet d'un coupe de torsion prédéterminé appliqué sur la susdite tige de préhension.

Cette vis de blocage, auto-taraudeuse et auto-cassable, permet de réduire les temps opératoires de la technique classique de mise en place d'une plaque d'ostéosynthèse, en réduisant le troisième et le quatrième stades de forage du trou et de torsion des vis, à une seule manipulation. En effet, une fois le modelage de la plaque exécuté, le chirurgien introduit la tige de préhension de la vis de l'invention à l'intérieur du porte-outil de l'arbre de sortie d'un moteur rotatif et applique la pointe de la tige filetée de ladite vis contre le tissu osseux du fragment de la fracture, via les ouvertures de ladite plaque, puis commande la rotation du porte-outil de manière à ce que ladite tige filetée, préférentiellement ménagée d'une entaille de perçage et de taraudage, s'enfonce en force dans le tissu osseux dans lequel elle est retenue. Dès que la vis est bloquée contre ladite plaque, le couple torsion appliqué sur la tige de préhension atteint un seuil minimum prédéterminé qui entraîne la rupture de celle-ci au niveau de la tête. L'intervention chirurgicale de mise en place de la plaque d'ostéosynthèse est alors terminée.

La triple fonction (auto-foreuse, auto-taraudeuse et auto-cassable) de la vis de l'invention, offre de nombreux avantages et parmi ceux-ci :
- l'absence de forets de perçage;
- l'absence d'un tournevis préhenseur;
- l'absence de vis de rattrapage évoquées ci-dessus et qui, présentant un diamètre plus gros, est utilisée à la suite d'un mauvais choix du foret en fonction de la nature de l'os ou lorsqu'un trou de forage est ovalisé par suite du fouettement du foret;
- l'obtention d'une tête de vis absolument intacte (le couple de torsion n'étant en effet appliqué que sur la tige de préhension) pour le démontage des plaques d'ostéosynthèse qui est préconisé au sixième mois suivant l'intervention.

Selon un mode de réalisation préférée de l'invention, la tige de préhension est de forme cylindrique et comporte au niveau de sa liaison avec la tête de vis, une encoche de fragilisation. Cette encoche de fragilisation est usinée dans la tige de préhension de manière à obtenir un étranglement dont la largeur et le diamètre définissent un seuil de rupture entre la tête de la vis et la tige de préhension, déterminé par la résistance maximale acceptée par le susdit étranglement sous l'effet d'un couple de torsion minimum appliqué sur la tige. Ce couple de torsion minimum sera bien évidemment fonction du couple de résistance de la pénétration de la tige filetée dans la masse du tissu osseux.

Selon une autre caractéristique particulièrement avantageuse de l'invention, le pas de la tige filetée d'ancrage de la vis est un pas présentant un angle pratiquement droit du type "pas d'artillerie" ou "pas canon", ledit pas offrant une meilleure résistance à l'arrachage notamment au niveau de l'os spongieux.

Selon une autre caractéristique particulièrement avantageuse de l'invention, la tête de la vis de blocage a une empreinte hexagonale. Ainsi, les pans des pourtours hexagonaux de la tête de la vis permettent d'utiliser un outil approprié lors de l'opération de dévissage. En effet , la face apparente de ladite vis ne peut comporter d'empreinte (cruciforme ou fente) puisqu'elle est, au départ, solidaire de la tige de préhension

L'invention a trait également à un procédé de fabrication d'une vis de blocage qui consiste :
- dans un premier temps, à décolleter une barre adoptant un profil hexagonal de manière à laisser sur une partie de sa longueur, une collerette hexagonale formant la tête de la vis, et à délimiter, de part et d'autre de ladite collerette, deux tiges cylindriques dont la longueur est égale pour la première à celle de la tige filetée de la vis et pour la deuxième à celle de la tige de préhension,
- dans un deuxième temps, à former, de préférence par roulage pour une meilleure finition du filet, un sillon hélicoïdal sur la surface cylindrique de la tige de la vis de manière à définir une tige filetée,
- dans un troisième temps, à préformer l'embout de la tige de préhension selon la définition du porte-outil de l'arbre de sortie du moteur rotatif,

- et dans un quatrième et dernier temps, à réduire l'autre tige cylindrique, au niveau de sa liaison avec la collerette hexagonale de la tête, de manière à obtenir un étranglement de largeur et de diamètre définissant le seuil de rupture entre la tête de la vis et celle de la tige de préhension sous l'effet d'un couple de torsion minimum appliqué sur la tige de préhension.

Ce procédé de fabrication est avantageux car il permet, à partir d'une barre hexagonale, de concilier toutes les caractéristiques innovantes de la vis de l'invention avec les critères de facilité de fabrication et de prix de revient réduit

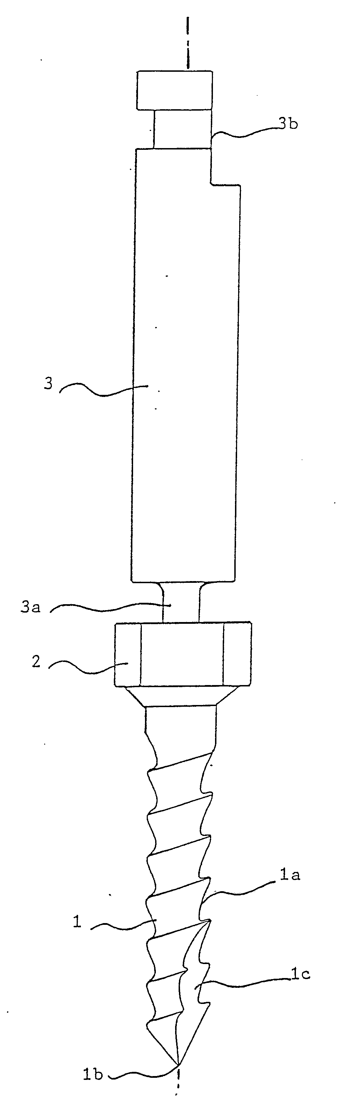

Bien que les concepts fondamentaux de l'invention considérés comme nouveaux aient été exprimés ci-dessus, de plus amples détails concernant un mode de réalisation préférée d'une vis de blocage respectant ces concepts fondamentaux, seront mieux compris en se référant à la description ci-après et au dessin l'accompagnant, illustrant par une vue de face, une telle vis

Telle que représentée sur ce dessin, la vis de blocage de l'invention est destinée à fixer une plaque d'un matériel d'ostéosynthèse apte à réunir par contention les fragments osseux d'une fracture.

Conformément aux dispositions principales de l'invention, elle se compose de trois parties bien distinctes :
- une tige 1 qui présente périphériquement une rainure hélicoïdale,
- une tête 2 au profil hexagonal,
- et une tige cylindrique de préhension 3.

La tige cylindrique 1 a un filetage 1a qui, obtenu de préférence par roulage, définit un pas présentant un angle pratiquement droit pour augmenter sa résistance à l'arrachage. Son embout libre est usiné de manière à définir une pointe 1b et une entaille de perçage et de taraudage 1c facilitant la pénétration de ladite tige dans le tissu osseux sous l'effet de la rotation de celle-ci.

La tête 2, solidaire de la tige filetée 1, a, quant à elle, un pourtour au profil hexagonal dont les pans viendront se loger à l'intérieur d'un outil de dévissage épousant les contours hexagonaux de ladite tête. Comme on peut le voir sur le dessin, la partie inférieure de ladite tête 2 est préformée coniquement afin de se caler dans les ouvertures de la plaque d'ostéosynthèse en épousant le fraisage intérieure de ces dernières.

La tige cylindrique 3, appelée dans le contexte de la présente invention tige de préhension, est préformée, au niveau de sa liaison avec la tête 2, d'un étranglement 3a obtenu par usinage de la tige 3 selon une largeur et un diamètre qui définissent un seuil de rupture entre la tête 2 et la tige 3, fonction de la résistance dudit étranglement sous l'effet d'un couple de torsion minimum appliqué sur la tige 3. Ainsi, dès que le couple de torsion appliqué sur la tige 3 sera supérieur à un seuil prédéterminé, une rupture au niveau de l'étranglement 3a permettra de dissocier la liaison entre la tige 3 et la tête 2.

Avantageusement, l'extrémité libre de la tige de préhension est ménagée d'un méplat 3b permettant de bloquer la position angulaire de la tige 3 à l'intérieur du porte-outil de l'arbre de sortie d'un moteur rotatif

Le procédé de fabrication de cette vis de blocage est simple et particulièrement indiqué pour une fabrication en série desdites vis. Selon ce procédé, une barre adoptant le profil hexagonal de la tête 2 est décolletée dans un premier temps de manière à laisser sur une partie de sa longueur, la collerette que forme la tête 2, et à délimiter, de part et d'autre de ladite collerette 2, les deux cylindres des tiges 1 et 3.

Dans un deuxième temps, un sillon hélicoïdal est formé, selon la méthode par roulage, sur la surface cylindrique de la tige 1 de manière à définir le filetage 1a de la sudite tige, lequel présente plutôt un pas à angle droit dit "pas d'artillerie" pour une meilleure tenue d'ancrage de la tige filetée 1 dans le tissu osseux. La pointe 1b et l'entaille de perçage 1c sont ensuite usinées selon des méthodes plus traditionnelles.

Dans un troisième temps, l'embout de la tige 3 est préformé notamment pour obtenir un méplat 3b permettant de caler la position de la tige 3 à l'intérieur du porte-outil de l'arbre de sortie du moteur rotatif.

Dans un quatrième et dernier temps, la tige cylindrique 3 est réduite au niveau de sa liaison avec la collerette hexagonale 2 de manière à obtenir un étranglement 3a de largeur et de diamètre prédéterminés en fonction des seuils de rupture à obtenir. Il est utile de préciser que ces seuils sont délimités par le coefficient de résistance à la pénétration de la vis, dans les différents tissus osseux.

On comprendra que la vis de blocage décrite ci-dessus ainsi que son procédé de fabrication ne l'on été qu'en vue d'une divulgation plutôt que d'une limitation et que de nombreuses modifications, combinaisons et substitutions peuvent être effectuées par l'homme de l'art sans s'éloigner ni de l'esprit ni de la portée de l'invention prise dans ses aspects les plus larges.

Afin de permettre une meilleure compréhension des dessins, une liste des références avec leurs légendes est ci-après énumérée

| 1 ..... | Tige filetée |
| 1a ..... | Sillon hélicoïdal de la tige 1 |
| 1b ..... | Pointe de la tige 1 |
| 1c ..... | Entaille de perçage de la tige 1 |
| 2 ..... | Tête hexagonale |
| 3 ..... | Tige cylindrique de préhension |
| 3a ..... | Etranglement de la zone de fragilisation |
| 3b ..... | Méplat de la tige 3. |

**Revendications**

1. Vis de blocage d'une plaque de matériel d'ostéosynthèse comprenant notamment :-
une tige cylindrique filetée (1) destinée à pénétrer dans le tissu osseux du fragment d'une fracture, via une des ouvertures ménagées dans ladite plaque,
- une tête (2) prenant appui sur la susdite plaque qu'elle maintient plaquée contre ledit fragment,
- et une tige de préhension cylindrique (3) associée à la tête (2) de ladite vis coaxialement à cette dernière pour être logée dans le porte-outil de l'arbre de sortie d'un moteur rotatif,
**CARACTERISEE PAR LE FAIT QUE** la susdite tige de préhension (3) comporte, au niveau de sa liaison avec la susdite tête de vis (2), un étranglement (3a) réduisant le diamètre de la tige de préhension (3) pour définir à cet endroit une zone de fragilisation rendant ladite tige (3) dissociable de la susdite tête (2) sous l'effet d'un couple de torsion minimum prédéterminé appliqué sur la susdite tige de préhension (3).

2. Vis de blocage selon la revendication 1, **CARACTERISEE PAR LE FAIT QUE** le pas de la tige filetée (1) d'ancrage de la vis est un pas présentant un angle pratiquement droit.

3. Vis de blocage selon la revendication 1, **CARACTERISEE PAR LE FAIT QUE** la partie inférieure de la tête (2) est préformée coniquement pour épouser les contours intérieurs du fraisage des ouvertures des plaques d'ostéosynthèse.

4. Vis de blocage selon la revendication 1, **CARACTERISEE PAR LE FAIT QUE** l'extrémité libre de la tige cylindrique de préhension (3) est ménagée d'un méplat (3b) pour caler la position angulaire de ladite tige à l'intérieur du porte-outil de l'arbre de sortie du susdit moteur rotatif.

5. Procédé de fabrication d'une vis de blocage selon les revendications 1 à 4 prises ensemble, **CARACTERISE EN CE QU'**il consiste :
- dans un premier temps, à décolleter une barre adoptant un profil hexagonal de manière à laisser sur une partie de sa longueur, une collerette hexagonale formant la tête (2) de la vis, et à délimiter, de part et d'autre de ladite collerette (2), deux tiges cylindriques (1 et 3) dont la longueur est égale pour la première à celle de la tige filetée (1) de la vis et pour la deuxième à celle de la tige de préhension (3),
- dans une deuxième temps, à former un sillon hélicoïdal (1a) sur la surface cylindrique de la tige (1) de la vis de manière à définir ladite tige filetée,
- dans un troisième temps, à préformer l'embout de la tige de préhension (3) selon la définition du porte-outil de l'arbre de sortie d'un moteur rotatif,
- et dans un quatrième et dernier temps, à réduire l'autre tige cylindrique (3), au niveau de sa liaison avec la collerette hexagonale de la tête (2), pour obtenir un étranglement (3a) de largeur et de diamètre définissant le seuil de rupture entre la tête (2) de la vis et la tige de préhension (3), qui est fonction de la résistance dudit étranglement (3a) sous l'effet d'un couple de torsion minimum appliqué sur la tige de préhension (3).

6. Procédé selon la revendication 5, **CARACTERISE EN CE QUE** le filetage (1a) de la tige décolletée (1) de la vis est obtenu par roulage.

7. Procédé selon les revendications 5 et 6, **CARACTERISE EN CE QUE** l'embout de la tige filetée (1) est usinée de manière à définir une pointe (1b) et une entaille de perçage et de taraudage (1c).

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y | FR-A-2 392 653  (JACQUET)<br>* page 1, lignes 25-30, page 2, lignes 15-25; revendications 1,6; figures 1,2 * | 1 | A 61 B  17/58 |
| Y | US-A-3 915 162  (MILLER)<br>* colonne 2, lignes 12-33; figure 5 * | 1 | |
| A | US-A-4 414 966  (STEDNITZ)<br>* colonne 3, ligne 39 - colonne 4; ligne 11; revendication 1; figures 1-4 * | 1,5-7 | |
| A | FR-A-2 347 027  (BIGGS)<br>* page 5, lignes 21-26; page 11; lignes 21-27; revendication 1; figures 1,14,16 * | 1 | |
| | ----- | | **DOMAINES TECHNIQUES RECHERCHES (Int. Cl.4)**<br><br>A 61 B  17/00 |

Le présent rapport a été établi pour toutes les revendications

| Lieu de la recherche | Date d'achèvement de la recherche | Examinateur |
|---|---|---|
| BERLIN | 07-03-1989 | MONNE E.M.B. |